# EUROPEAN PATENT SPECIFICATION

(11) **EP 4 236 771 B1**
(45) Date of publication and mention of the grant of the patent: **12.03.2025**
(21) Application number: 21887375.0
(22) Date of filing: 26.10.2021
(51) Int. Cl.: A61B 5/00, A61B 18/14, C09K 11/06, G01N 27/327

(54) **HIGH-TRANSCONDUCTANCE ORGANIC ELECTROCHEMICAL TRANSISTOR (OECT)-BASED SENSORS AND METHODS OF USE**
SENSOREN AUF BASIS ORGANISCHER ELEKTROCHEMISCHER TRANSISTOREN MIT HOHER TRANSKONDUKTANZ UND VERFAHREN ZUR VERWENDUNG
CAPTEURS À BASE DE TRANSISTOR ÉLECTROCHIMIQUE ORGANIQUE (OECT) À TRANSCONDUCTANCE ÉLEVÉE ET PROCÉDÉS D'UTILISATION

(30) Priority: 30.10.2020 US 202063108176 P
(43) Date of publication of application: 06.09.2023
(73) Proprietor: The Regents of University of California, Oakland, CA 94607-5200 (US)
(72) Inventor: KHADEMHOSSEINI, Alireza, Los Angeles, California 90095-7191 (US); ZHANG, Shiming, Los Angeles, California 90095-7191 (US)
(74) Representative: Ipsilon Benelux
(86) International application number: PCT/US2021/056712
(87) International publication number: WO 2022/093866

(56) References cited:
- WO-A1-2019/224628
- US-A- 5 147 737
- US-A1- 2017 000 368
- US-A1- 2019 381 503
- US-A1- 2020 072 780
- US-A1- 2020 138 343
- PECQUEUR S ET AL: "Concentric-electrode organic electrochemical transistors: case study for selective hydrazine sensing", ARXIV.ORG, CORNELL UNIVERSITY LIBRARY, 201 OLIN LIBRARY CORNELL UNIVERSITY ITHACA, NY 14853, 22 July 2017 (2017-07-22), XP081278905, DOI: 10.1088/1742-6596/939/1/012017

## Description

### Related Application

This Application claims priority to U.S. Provisional Patent Application No. 63/108,176 filed on October 30, 2020. Priority is claimed pursuant to 35 U.S.C. § 119 and any other applicable statute.

### Technical Field

The technical field relates to organic electrochemical transistor (OECT)-based sensors. More specifically, the technical field relates to OECT-based sensors that achieve high-transconductance that use a circular and wavy channel geometry. The sensor design may be used for multiplexed OECT-based biosensors for high-throughput biomarker screening applications.

### Statement Regarding Federally Sponsored

### Research and Development

This invention was made with government support under Grant Number GM126571, awarded by the National Institutes of Health. The government has certain rights in the invention.

### Background

Biomarkers are molecules that can be detected as indicators of disease processes and body responses to therapeutic intervention. Enzyme-linked immunosorbent assay (ELISA) is currently the gold standard to measure biomarkers. ELISA uses a highly-specific antibody to capture the antigen (capturing antibody). Then the captured antigen needs to be further linked to a secondary detecting antibody for quantification. The multiple operation steps make it challenge to be widely used for point-of-care (POC) applications. A growing trend now is to develop multiplexed immunosensors for high-throughput biomarker screening in one POC device. Such a device will provide more comprehensive information to the caregivers for better disease management. Such POC screening devices are could be used for early detection and monitoring of cancer and other immune-related diseases such as cancer immunotherapy and acute respiratory distress syndrome.

Electrochemical sensors are considered competitive candidates for POC applications. To further improve the sensitivities and lower the limits of detection, electrochemical sensors must be assisted with amplification strategies, often based on enzymatic labeling on the detecting antibody. OECTs have been proved to be efficient strategy to amplify the electrochemical signal. OECT owns the highest transconductance (amplification ability) due to its unique mixed ion/electron conduction property and bulk-modulation ability of the channel. It is now clear that OECT is a superior technique for developing highly-sensitive electrochemical sensors. For example, OECT demonstrates superior signal-to-noise ratio in detecting brain signals. See Khodagholy et al., In vivo recordings of brain activity using organic transistors, Nature Communications, 4:1575 (2013). Besides, OECT can be easily mass-produced with all solution-processable and printable techniques on cost-effective plastics. These advantages make OECT competitive for commercial applications. Currently, examples have been demonstrated with OECT for enzymatic sensors and immunosensors. However, multiplexed OECT immunosensors for high-throughput detection of biomarkers with capturing antibodies or aptamers have not been demonstrated. Such a multiplexed OECT-based immunosensing platform will revolutionize the current POC immunosensors markets regarding sensitivity, detection limit, and cost-effectiveness. PECQUEUR S ET AL: "Concentric-electrode organic electrochemical transistors: case study for selective hydrazine sensing" is relevant prior art.

### Summary

In one embodiment, an organic electrochemical transistor (OECT)-based biosensor device is disclosed. The OECT-based sensor comprises a substrate, a gate electrode disposed on the substrate and having capture agents disposed thereon, the capture agents are specific to an antigen (e.g., biomarker/biomolecule), and source and drain electrodes disposed on the substrate and at least partially surrounding the gate electrode, the source and drain electrodes forming an undulating pattern around the gate electrode and separated from one another by a channel. Optionally blocker or spacer molecules may be located on the gate electrode.

In one embodiment, a multiplexed OECT-based biosensor for high-throughput biomarker screening is disclosed. The sensor uses a circular and wavy (CW) channel structure to obtain high-transconductance OECT (HT-OECT). The design of multiplexed HT-OECT biosensors is accomplished by assembling multiple HT-OECT sensors together with multiple capture agent-modified gate electrodes on the same microfluidic chip (or substrate). Such multiplexed HT-OECT biosensors can be used for high-throughput biomarker screening for a better disease monitoring and treatment. Combined with portable device
characterization systems, the HT-OECT multiplexed biosensor can be used for POC applications, wearable applications on the skin, and minimally-invasive biosensing applications using microneedle and catheter as minimally invasive carriers.

In one embodiment, an organic electrochemical transistor (OECT)-based biosensor device includes a substrate and a gate electrode disposed on the substrate or a separate substrate and having capture agents disposed thereon, the capture agents specific to a biomarker. The device further includes source and drain electrodes disposed on the substrate and at least partially surrounding the gate electrode, the source and drain electrodes forming an undulating pattern around the gate electrode and separated from one another by a channel.

In one embodiment, a multiplexed organic electrochemical transistor (OECT)-based biosensing device includes a substrate and a plurality of organic electrochemical transistor (OECT) sensors disposed on the substrate, wherein the plurality of organic electrochemical transistor (OECT) sensors are specific to different antigens (e.g., biomarkers/biomolecules). Each organic electrochemical transistor (OECT) sensor includes a gate electrode disposed on the substrate (or a separate substrate) and having capture agents disposed thereon, the capture agents specific to the different antigens. Source and drain electrodes are disposed on the substrate and at least partially surrounding the gate electrode, the source and drain electrodes forming an undulating pattern that is generally described as circular and wavy (CW) around the gate electrode and separated from one another by a channel.

In another embodiment, a wearable organic electrochemical transistor (OECT)-based biosensor device includes a flexible or soft substrate comprising one or more organic electrochemical transistor (OECT) sensors disposed on the flexible or soft substrate. Each organic electrochemical transistor (OECT) sensor includes a gate electrode disposed on the flexible or soft substrate (or a separate flexible or soft substrate) and having capture agents disposed thereon, the capture agents specific to antigens (e.g., biomarkers/biomolecules). Source and drain electrodes are disposed on the substrate and at least partially surrounding the gate electrode, the source and drain electrodes forming an undulating pattern (e.g., CW configuration) around the gate electrode and separated from one another by a channel. The wearable (OECT)-based biosensor device may, in some embodiments, include microneedles that penetrate the skin or other tissue and absorb bodily fluid which is then exposed to the one or more sensors. In other embodiments, one or more inlets are provided in which sweat or other bodily fluids can enter and then flow over the one or more sensors.

In another embodiment, a minimally invasive catheter or probe device for detecting biomarkers includes an elongate catheter or shaft having one or more organic electrochemical transistor (OECT)-based biosensor(s) mounted thereon, the biosensor(s) comprising a substrate and a gate electrode disposed on the substrate and having capture agents disposed thereon, the capture agents specific to one or more biomarkers. Source and drain electrodes are disposed on the substrate and at least partially surrounding the gate electrode, the source and drain electrodes forming an undulating pattern (e.g., CW configuration) around the gate electrode and separated from one another by a channel.

To use the organic electrochemical transistor (OECT)-based biosensor device, a fluid (e.g., bodily fluid) is exposed to the sensor(s). The fluid may be input into the device or absorbed (e.g., using minimally invasive microneedles). The current response of the sensor is monitored (e.g., source-drain current (I_{ds})) to detect the presence of (or concentration) of one or more biomarkers/biomolecules. A Source Measuring Unit (SMU) may be used for this purpose. The sensor may be embodied as a microfluidic chip or flow cell. In other embodiments, the sensor is wearable and may be made flexible or soft. In other embodiments, the sensor is integrated in with a patch containing microneedles, a catheter or a probe device for minimally invasive applications. Example bodily fluids that can be measured include blood and urine.

### Brief Description of the Drawings

FIG. 1A schematically illustrates a conventional (prior art) OECT.
FIG. 1B illustrates an embodiment of a HT-OECT biosensor device with source and drain electrodes in a curvy and wavy (CW) configuration used to increase the W/L ratio for higher transconductance. A channel with the CW configuration surrounds the gate electrodes and is favorable for uniform doping/de-doping of the PEDOT:PSS channel. The gate is modified with antibody (or other capture agents) for capturing specific antigen(s).
FIG. 1C illustrates the chemical structure of poly(3,4-ethylenedioxythiophene) polystyrene sulfonate (PEDOT:PSS) which is used in the channel of the HT-OECT sensor.
FIG. 2 schematically illustrates how the circular gate electrode is modified with a capture agent (e.g., antibody) for capturing antigens along with blockers to minimize the non-specific binding to the gate electrode.
FIG. 3 illustrates a HT-OECT biosensor device being electrically connected to external circuitry used to operate the HT-OECT biosensor device. Voltages may be applied to the biosensor device and current response measured using a Source Measure Unit (SMU) such as the B2902A Precision Source/Measure Unit (available Keysight Technologies). Of course, this is only one example of a SMU.

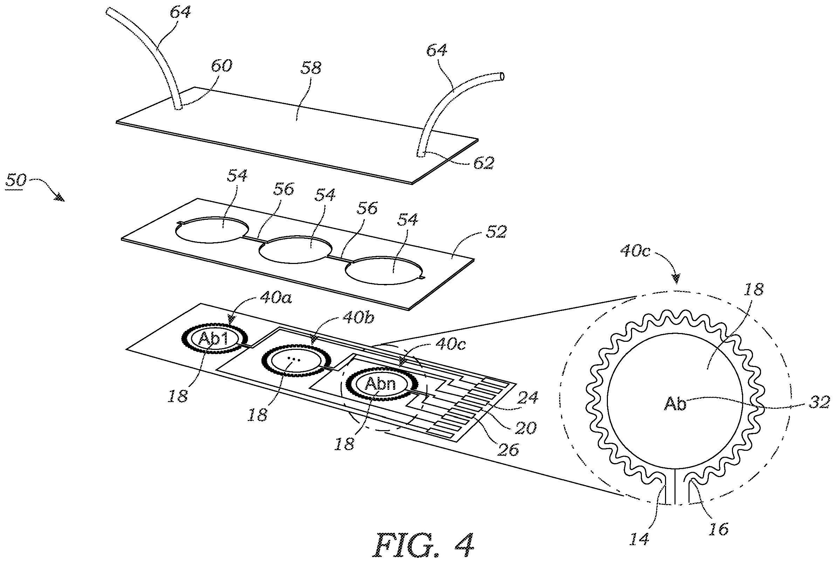

FIG. 4 illustrates one embodiment of a multiplexed HT-OECT biosensor device that is disposed on a substrate or on-chip (e.g., a microfluidic chip). The HT-OECT sensing elements (e.g., individual sensors) are first fabricated on a chosen substrate. In the HT-OECT sensing elements, each gate electrode is modified with an antibody (or other capture agent) that captures a specific antigen. Different gate electrodes are modified with different antibodies for different antigen capturing and sensing. The array is then be sandwiched with a cover or top layer through an intermediate PDMS layer with microfluidic channels and chambers that provide a flow path between an inlet and outlet in the microfluidic chip. In alternative embodiments, the gate electrodes could also be fabricated and modified with antibody on the underside of cover or top layer. Fluid containing the antigen(s) is introduced into the PDMS microchannels via an inlet and contacts the various sensing elements. The HT-OECT biosensor device can then be interrogated with external circuitry to detect and/or measure the concentrations of different antigens.
FIG. 5A illustrates an embodiment of wearable multiplexed HT-OECT device in the form of a patch that has a plurality of microneedles extended away from a base or substrate.
FIG. 5B illustrates the wearable multiplexed HT-OECT device of FIG. 5A being placed onto the skin of a subject.
FIG. 6 illustrates an embodiment of a catheter or probe device that incorporates one or more HT-OECT sensors thereon. The sensors may be disposed on a distal end of the catheter or probe and can be used to detect and/or measure the concentration of antigens in bodily fluid (e.g., blood, urine, brain cerebrospinal fluid).
FIG. 7A illustrates a graph showing impedance changes in the HT-OECT biosensor device (without a capture agent) for the detection of codeine. The gate electrode modified with blocker (but without antibody) shows no impedance change to the concentration change of the antigen codeine.
FIG. 7B illustrates a graph showing impedance changes in the HT-OECT biosensor device (with a capture agent) for the detection of codeine. The gate electrodes modified with both blocker and antibody shows clear impedance change to the concentration change of codeine.
FIG. 7C illustrates a graph showing the change in drain-source current I_{ds} as a function of time. The HT-OECT biosensor device shows a steeper decrease in I_{ds} at increased codeine concentrations. This indicates that the rate of change in I_{ds} can be used to measure the concentration of the antigen in the fluid exposed to the HT-OECT biosensor device.

### Detailed Description of Illustrated Embodiments

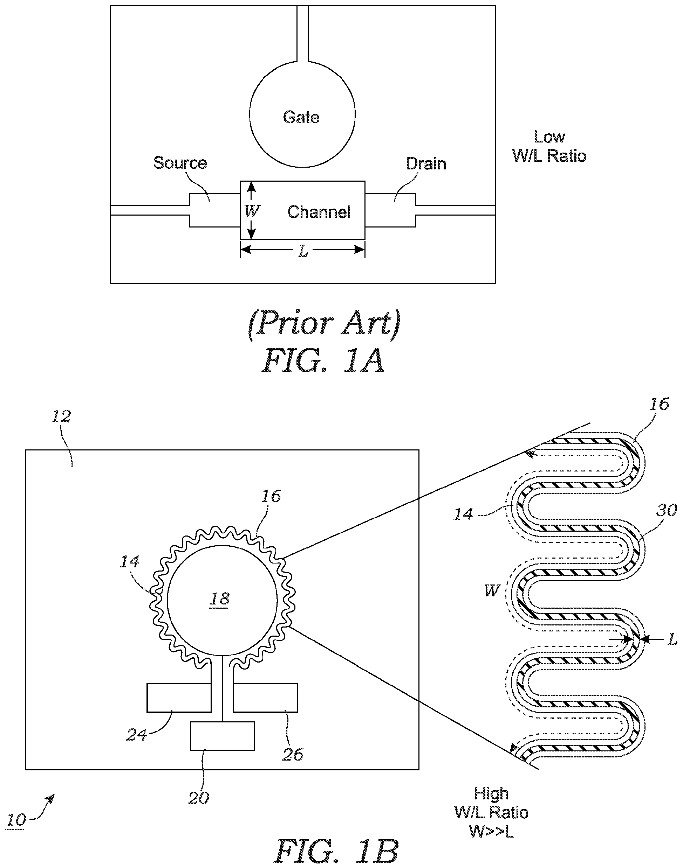

FIG. 1B schematically illustrates an organic electrochemical transistor (OECT)-based biosensor device 10 according to one embodiment. The OECT-based biosensor device 10 includes a substrate 12 on which the source electrode 14 and drain electrode 16 is formed. In addition, in some embodiments, the gate electrode 18 of the OECT-based biosensor device 10 is also formed on the substrate 12. In other embodiments, the gate electrode 18 is formed on a secondary substrate 20 which is located adjacent to or in close proximity with the substrate 12. For example, as explained herein, the substrate 12 that includes the source electrode 14 and the drain electrode 16 may form the bottom surface of the OECT-based biosensor device 10 while the secondary substrate 20 that includes the gate electrode 18 may form the top of the OECT-based biosensor device 10 (or vice versa). The substrate(s) 12, 20 may be rigid or flexible or soft. Examples include glass, plastics, elastomers, hydrogels, and other biocompatible polymers. For soft materials used as part of the substrate 12, 20, it is preferrable to use soft materials with low oxygen permeability such as thermoplastic polyurethane (TPU), which can contribute to a higher doping/de-doping efficiency of the channel 30 on the soft substrate 12, 20.

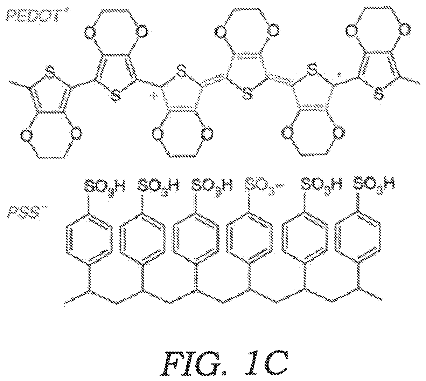

As seen in FIG. 1B, the source electrode 14 is formed on the substrate 12 and includes an undulating pattern that meanders back-and-forth in a curved and wavy pattern about the periphery of the gate electrode 18 (e.g., in a partial circular or arcuate path around the central gate electrode 18). In FIG. 1B, the gate electrode 18 centrally located within respect to the undulating portion of the source electrode 14 and the drain electrode 16. The gate electrode 18 is circular shaped as seen in FIG. 1B and is electrically connected to a contact pad 22 via a conductive trace, line, or wire. Similarly, the source electrode 14 is electrically connected to a contact pad 24 via a conductive trace, line, or wire. The drain electrode 16 is electrically connected to a contact pad 26 via a conductive trace, line, or wire. The channel 30 of the OECT-based biosensor device 10 is located between the undulating portion of the source electrode 14 and the drain electrode 16. This is best seen in the enlarged view of FIG. 1B. The channel 30 is formed between the source electrode 14 and drain electrode 16 and may include poly(3,4-ethylenedioxythiophene) polystyrene sulfonate (PEDOT:PSS) (FIG. 1C). Additives such as dodecylbenzenesulphonic acid (DBSA), glycerol, ethylene glycol (EG), a fluorosurfactant (e.g., ZONYL^{®}), (3-glycidyloxypropyl)trimethoxysilane (GOPS) may be added into PEDOT:PSS to control its properties. Thus, the channel 30 also at least partially surrounds the gate electrode 18 and has a similar undulating configuration as the source electrode 14 and the drain electrode 16 and is favorable for uniform doping/de-doping. As seen in FIG. 1B, by having the source electrode 14 and drain electrode 16 arranged in this curved and wavy configuration with the channel 30 formed between, the OECT-based biosensor device 10 has an increased W/L ratio for higher transconductance. The distance between the source electrode 14 and drain electrode 16 (L) is small while the distance along the width (W) is much larger (i.e., W>>L). This produces the higher W/L ratio for higher transconductance. This is in contrast with the prior art design illustrated in FIG. 1A which has a low W/L ratio. The distance between the source electrode 14 and drain electrode 16 (L) is substantially uniform along the length of the source electrode 14 and drain electrode 16. Exemplary dimensions (e.g., diameter) of the gate electrode 18 range from 1 µm to 1 cm, although a preferred embodiment may have the gate electrode 18 with a diameter of several millimeters. The dimensions of the source electrode 14 and drain electrode 16 depends on the size of the gate electrode 18. An exemplary dimension for the source electrode 14 and drain electrode 16 includes a length of around 66 mm and a width of around 500 µm. Exemplary dimensions of the channel 30 include a length of around 500 µm and a width of around 66 mm.

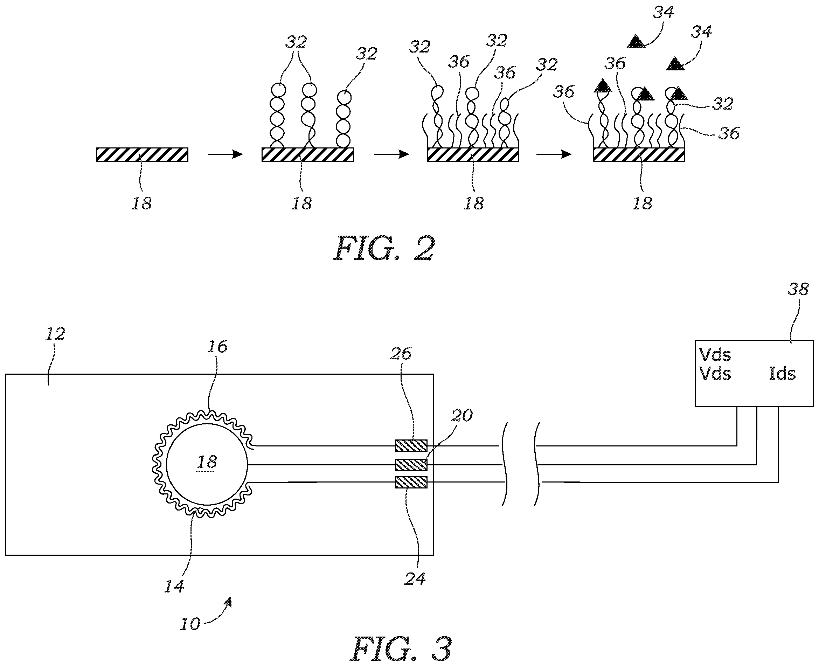

With reference to FIG. 2, the gate electrode 18 is modified or functionalized with capture agents 32 (e.g., antibodies, aptamers, nucleic acids) for capturing specific antigen(s) 34. The antigens 34 may include particular analytes, biomolecules, or biomarkers. In one preferred embodiment, the antigens 34 include biomolecules or biomarkers that are immunological biomolecules or biomarkers. As one example, the biomarkers may include interferons or interleukins. Examples include, for instance, the biomarkers IFN-λ, thrombopoietin, IL-21, IL-23, and IL-33 which can be used to diagnose the severity of COVID-19 patients. The biosensor device 10 thus operates as an immunosensor. Of course, the invention is not so limited and may include any type of antigen 34. The gate electrode 18 may, in some embodiments, include a spacer or blocker molecule 36 to minimize the non-specific binding of antigen. Examples of the spacer or blocker molecule 36 include bovine serum albumin (BSA) and 6-mecapto-1-hexanol (MCH).

With reference to FIG. 3, the biosensor device 10 includes external circuitry 38 that is configured to apply a gate-source voltage and a drain-source voltage and measure the drain-source current. The source-drain voltage (V_{ds}) is preferably a fixed DC value. The gate-source voltage (V_{gs}) may be constant, swept, or applied at a certain frequency (either DC or AC). DC is preferred but AC may be used. By monitoring the source-drain current (I_{ds}), one can monitor for the presence (or absence) of antigens 34 (e.g., biomolecules or biomarkers). The rate of change of the source-drain current (I_{ds}) may also be monitored to determine the concentration of antigens 34. Voltages may be applied to the biosensor device 10 and current response measured using a Source Measure Unit (SMU) such as the B2902A Precision Source/Measure Unit (available Keysight Technologies). For example, a single SMU 38 may be used to apply voltages and measure current for multiple sensing elements on a multiplexed biosensor device 50 such as that illustrated in FIG. 4.

In one embodiment, and with reference to FIG. 4, a multiplexed biosensor device 50 is provided that incorporates multiple sensing elements 40a, 40b, 40c thereon with different sensing elements 40a, 40b, 40c tailored to different antigens 34. In this embodiment, a substrate 12 (e.g., microfluidic chip) is provided that has a plurality of organic electrochemical transistor (OECT) sensing elements 40a, 40b, 40c disposed thereon/therein, wherein the plurality of organic electrochemical transistor (OECT) sensing elements 40a, 40b, 40c are specific to different antigens 34. Each organic electrochemical transistor (OECT) sensing elements 40a, 40b, 40c includes a gate electrode 18 disposed on the substrate 12 and having capture agents 32 disposed thereon, the capture agents 32 specific to the different antigens 34. A source electrode 14 and drain electrode 16 (for each sensing element 40a, 40b, 40c) are disposed on the substrate 12 and at least partially surround the gate electrode 18 as described herein, the source and drain electrodes 14, 16 forming an undulating pattern around the respective gate electrode 18 and separated from one another by a channel 30.

A multi-layer structure may be formed as illustrated in FIG. 4 to create a complete microfluidic chip device 50 that acts as a flow cell device in which a sample flows through the microfluidic chip device 50. The microfluidic chip device 50 includes an intermediate layer 52 that forms or defines devices microfluidic chambers 54 and channels 56. The chambers 54 surround the gate electrodes 18 of the sensing elements 40a, 40b, 40c and channels 54 allow for fluid to flow across each of the sensing elements 40a, 40b, 40c to expose the fluid to the respective gate electrodes 18. The microfluidic chip device 50 includes a top or capping layer 58 that includes an inlet 60 and outlet 62. Tubing 64 may be coupled to the inlet 60 and outlet 62 and is used to deliver a fluid sample to the microfluidic chip device 50 as well as evacuate fluid from the microfluidic chip device 50. Fluid can be delivered to the microfluidic chip device 50 manually or using a pump device. Fluid may be continuously pumped through the microfluidic chip device 50 or, alternatively, fluid may be flowed into the microfluidic chip device 50 and stopped to expose the fluid to the sensing elements 40a, 40b, 40c for a period of time to allow binding/hybridization and signal generation. In some embodiments, the sensing elements 40a, 40b, 40c may be used repeatedly to detect or measure antigens 34 in a sample. For instance, antigens 34 that are bound to the capture agents 32 (such as antibody Ab of FIG. 4) may be liberated back into the surrounding fluid using a chemical agent or the like to strip adhered antigens 34 from the gate electrode(s) 18. In some embodiments, the top or capping layer 58 serves as the secondary substrate 20 and holds the gate electrode(s) 18. In this embodiment, the gate electrode(s) 18 is/are located on the secondary substrate 20 (e.g., on the side of the secondary substrate 20 that faces the source/drain electrodes 14, 16) while the source electrode(s) 14 and drain electrode(s) 16 are located on the "main" substrate 12. The gate electrode 18 for each sensing element 40a, 40b, 40c is positioned adjacent to the respective source electrode(s) 14 and drain electrode(s) 16 as explained herein.

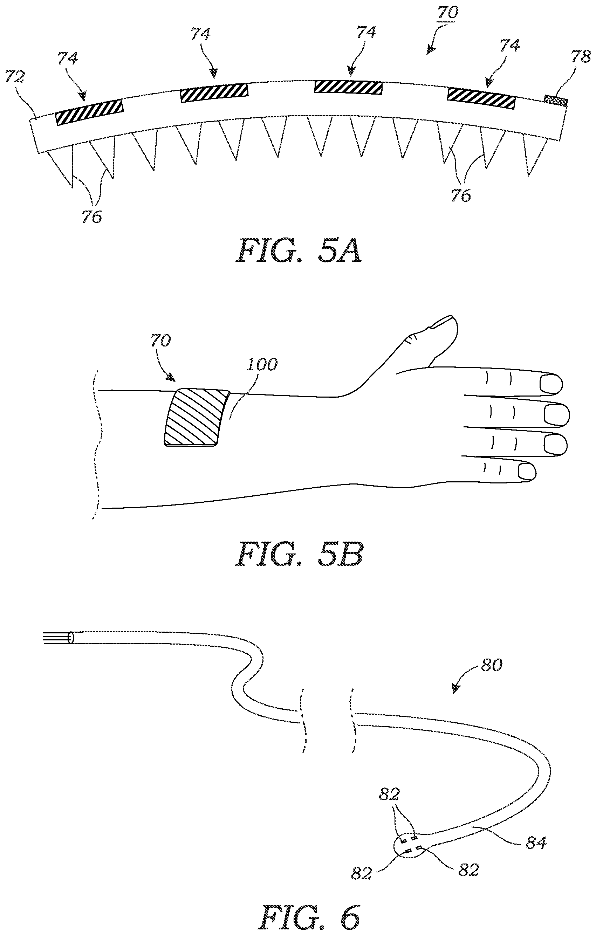

In another embodiment, and with reference to FIGS. 5A and 5B, a wearable organic electrochemical transistor (OECT)-based biosensor device 70 is disclosed that can be worn on the skin of the subject (or other tissue). The biosensor device 70 includes a flexible substrate 72 comprising one or more organic electrochemical transistor (OECT) sensors 74 disposed on the flexible substrate 72, the flexible substrate 72 having one or more organic electrochemical transistor (OECT) sensors 74. Each organic electrochemical transistor (OECT) sensor 74 is similar to the construction used in the biosensor device 10 described previously. Namely, each sensor 74 includes a gate electrode 18 disposed on the flexible substrate 72 and having capture agents 32 disposed thereon, the capture agents 32 specific to a particular antigen 34. Each sensor 74 may sense the same antigen 34 or, alternatively, different sensors 74 sense different antigens 34. Source and drain electrodes 14, 16 are disposed on the flexible substrate 72 and at least partially surrounding the gate electrode 18, the source and drain electrodes 14, 16 forming an undulating pattern around the gate electrode 18 and separated from one another by a channel 30.

In this embodiment, the flexible substrate 72 is made from a biocompatible polymer such as a hydrogel material that allows for the passage of fluid and chemical species through the material of the flexible substrate 72 and in contact with the sensors 74. This may include, for example, materials such as poly (methyl methacrylate) (PMMA), polylactic acid (PLA), poly (lactic-co-glycolic acid) (PLGA), polyglycolic acid (PGA), poly (carbonate), cyclic-olefin copolymer, poly (vinylpyrrolidone) (PVP), poly (vinyl alcohol) (PVA), polystyrene (PS) poly (methyl vinyl ether-co-maleic anhydride), and gelatin methacryloyl (GelMA). The biosensor device 70 includes a plurality of microneedles 76 that extend generally orthogonally from the flexible substrate 72. The microneedles 76 may be conical, pyramidal, or similar shape that terminate at a tip. The microneedles 76 may have a length of less than about 1 mm and more preferably several hundred micrometers and are able to penetrate the tissue 100 of a subject. Thus, the biosensor device 70 may be worn like a patch, bandage or the like as seen in FIG. 5B. The microneedles 76 that penetrate the subject's skin or other tissue 100 absorb or transfer bodily fluids (e.g., interstitial fluids) to one or more sensors 74 in the biosensor device 70. The microneedles 76 thus act as functional inlets to the biosensor device 70 and allow flow and antigen(s) to enter the flexible substrate 72 and contact the sensors 74. For example, as one example, the wearable biosensor device 70 may include sensors 74 used to measure glucose levels in the subject via interstitial fluids measurements that are accessed using microneedles 76.

The wearable biosensor device 70 may include electrical contacts 78 that are used to connect with external circuitry 38 to measure the response of the sensors 74. For example, the wearable biosensor device 70 may be periodically interrogated with external circuitry 38 that can be temporarily connected to the wearable biosensor device 70 via the electrical contacts 78. Alternatively, external circuitry 38 may be permanently connected to the wearable biosensor device 70.

In another embodiment, a minimally invasive catheter or probe device 80 incorporates one or more (OECT)-based sensors 82 for detecting antigens 34. The device includes 80 an elongate catheter or shaft 84 having one or more organic electrochemical transistor (OECT)-based sensor(s) 82 mounted thereon, the sensor(s) 82 including a substrate 12 (which may be a surface of the catheter or shaft 84) and a gate electrode 18 disposed on the substrate 12 and having capture agents 32 disposed thereon, the capture agents 32 specific to one or more antigens 34. Source and drain electrodes 14, 16 are disposed on the substrate 12 and at least partially surround the gate electrode 18, the source and drain electrodes 14, 16 forming an undulating pattern around the gate electrode 18 and separated from one another by a channel 30 as described previously. Electrically traces, lines, or wires 86 may extend proximally form the source electrodes 14, drain electrodes 16, and gate electrodes 18 through the catheter or shaft 84 which are then coupled to external circuitry 38 to apply the voltages to the sensor(s) 82 and monitor response. The catheter or probe device 80 may be used intravascularly in the cardiovascular system. The catheter or probe device 80 may also be used to traverse the urethra to detect and/or measure antigens 34 in urine. In addition, the catheter or probe device 80 could be incorporated into a probe device 80 that is used as a brain probe.

The fluid that may come into contact with the biosensor device 10, 50, 70, 80 may be a physiological fluid such as blood, urine, interstitial fluid, or the like. The fluid may enter the device 10, 50, 70, 80 via one or more inlets 60 or microneedles 76 in other embodiments. Alternatively, the sensing surface of the sensor device 80 may be exposed directly to the biological fluid. The electrical response of the device 10, 50, 70, 80 is then monitored or analyzed as the fluid is run through the sensor device 10, 50 or otherwise exposed to the device 70, 80. This includes applying a gate-source voltage and a drain-source voltage and measuring the drain-source current with the circuitry 38. The substrate 12 may include electrical contacts or pads 22, 24, 26 as explained herein to interface with respective gate electrode 18, source electrode 14, and drain electrode 16. For example, leads or wires can connect the external circuitry 38 to the sensor device 10, 50, 70, 80. Alternatively, such electronics could be integrated on-chip/on-substrate or adjacent thereto in some embodiments.

### Experimental

### Results and discussions

The transconductance of an OECT represents its amplification ability. Higher transconductance contributes to higher sensitivity and lower detection limits. The transconductance can be increased by increasing the W/L ratio and thickness of the channel 30. The W/L ratio can be increased by changing the geometry of the device. Interdigitated electrodes (IDE) have been demonstrated to increase the W/L ratio in a limited area. To increase to the W/L, at the same time considering the uniformity modulation of the gate electrode 18, the biosensor device of FIG. 1B was created. The source electrode 14 and drain electrode 16 surround the circular gate electrode 18. Compared to conventional IDE, this design is favorable for a more uniform doping/doping of the channel 30 at a given gate potential. The undulating or wavy structure of the source electrode 14 and drain electrode 16 is also favorable to increase the mechanical flexibility and stretchability of the device 10. Compared to the commercial electrochemical sensing electrodes, the W/L ratio can be increased by several orders of magnitudes, which indicates a much higher amplification ability of the biosensor device 10, thus enables a higher sensitivity and a lower detection limit.

First, the gate electrode 18, the source electrode 14, and the drain electrode 16 of HT-OECT biosensor device 10 were fabricated on a glass substrate 12 (but can be plastics and elastomers). Afterward, CW PEDOT:PSS channel 30 is patterned between the source electrode 14 and drain electrode 16 with photolithographic technique. The Au gate electrode 18 is then modified with capturing antibodies 32 with high affinity for the targeted biomarker (antigen 34, FIG. 2). As a demonstration, the use of the HT-OECT biosensor device 10 was shown for the detection of codeine, a pain reliever small molecular drug that is sometimes used by immunotherapy patients. The codeine-antibody 32 on gate electrode 18 was modified. The thiol group in the antibody 32 improved the bonding with the Au electrode 18. The gate electrode 18 is then further treated with spacers or blockers 36 such as 6-mecapto-1-hexanol (MCH) and bovine serum albumin (BSA) to minimize the non-specific binding of the antigen codeine to its surface.

A PDMS microfluidic chip device 50 such as that illustrated in FIG. 4 includes a microfluidic channel 56 is then fabricated to guide the flow to the HT-OECT sensing element (e.g., a flow cell or microfluidic chip is formed). Finally, a top or capping layer 58 is used to seal the microfluidic channel 56. The codeine-antibody modified gate electrode 18 may also be fabricated on the capping or top layer 58. In this case, the assembly of HT-OECT biosensor device 10 is completed by aligning and sandwiching the top or capping layer 58 (w/ gate electrode 18) and the substrate 12 (w/ source and drain electrodes 14, 16) through the PDMS microfluidic channels 56.

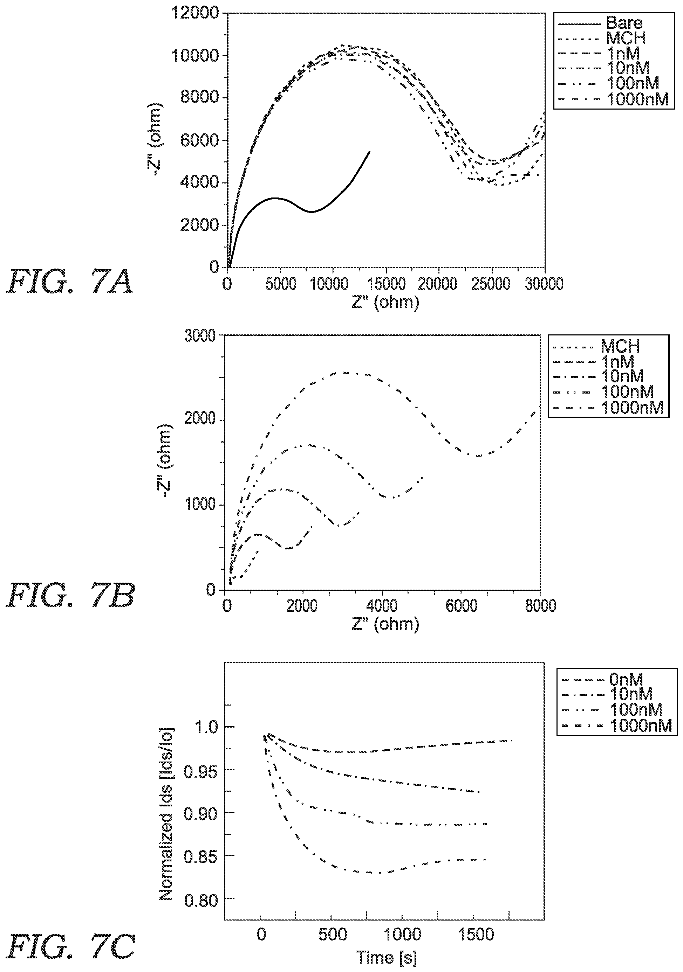

To detect the codeine with HT-OECT biosensor device 10. The gate voltage (V_{gs}) may be constant, swept, or applied at a certain frequency, while the source-drain voltage (V_{ds}) is fixed constant. FIG. 7A illustrates a graph showing impedance changes in the HT-OECT biosensor device 10 (without a capture agent 32) for the detection of codeine. The gate electrode 18 modified with spacer/ blocker 36 (but without antibody) shows no impedance change to the concentration change of the antigen codeine. FIG. 7B illustrates a graph showing impedance changes in the HT-OECT biosensor device 10 (with a capture agent 32) for the detection of codeine. The gate electrodes 18 modified with both spacer/blocker 36 and antibody 32 shows clear impedance change to the concentration change of codeine. When there is no antibody-antigen (codeine) binding, the source-drain current (I_{ds}) maintains a stable value (FIG. 7C). When the antigen 34 (i.e., codeine) is present in the fluid, it binds with the antibody 32, which increases the gate/electrolyte capacitance and finally decreases the I_{ds}. The I_{ds}-decrease profile is dependent on the concentration of the antigen codeine. A steeper decrease in I_{ds} with time indicates a higher codeine concentration in the fluid. As shown in FIG. 7C, increasing the codeine concentration in the fluid causes a steeper decrease in the I_{ds}, demonstrating the potential of the HT-OECT biosensor device 10 in detecting the antigen codeine.

The demonstrated HT-OECT biosensor device 10 may be scaled up for multiplexed HT-OECT immunosensors. The design is illustrated in FIG. 4. In this design, a number of gate electrodes 18 are introduced on the same microfluidic chip device 50 (e.g., chip) and modified with capturing antibodies 32 for the detection of different antigens 34. The number of gate electrodes 18 depends on the number of antigens 34 required to monitor the disease. For example, mild or severe COVID-19 patients can be diagnosed by measuring the following five biomarker antigens 34 IFN-λ, thrombopoietin, IL-21, IL-23, and IL-33. In this case, corresponding five multiplexed HT-OECT-based sensing elements 40 can be fabricated on the same substrate 12 for high-throughput biomarker screening. The detection of more biomarkers can be realized by increasing the number of OECT sensing elements 40 integrated on the microfluidic chip device 50. Because the antibody is highly specific to a certain biomarker or antigen 34, the presence of other antigens 34 or biomarkers in the sample will have little influence on other HT-OECT-based sensing elements 40.

The design of multiplexed HT-OECT biosensor devices 10 can be used for POC tests, wearable biosensing, and minimally-invasive biosensing by using microneedles 76 (FIGS. 5A, 5B), catheter and/or probe (FIG. 6) as carriers for the sensors 74, 82. In particular, the multiplexed HT-OECT can be developed as ion sensors, enzymatic sensors, electrolyte sensors, metabolites or metal ion sensors on microneedles and catheters, which have not been reported so far.

First, the design of multiplexed HT-OECT biosensor device 10 can be used for the POC tests. For example, with reference to the embodiment of FIG. 4, the collected bodily fluid can be pumped or input into the inlet 60 of the microfluidic chip device 50. The flow is enabled either by external pressure or capillary force. Once antigens 34 (e.g., biomarkers) in the flow pass the corresponding antibody-modified gate electrodes 18, they are captured. The binding process increases the gate/electrolyte capacitance. The change in the capacitance leads to the change in the effective V_{gs} and finally results in a more pronounced change in the I_{ds} of the HT-OECT which is detected or measured by external circuitry 38. Further integration with a portable data readout system allows the simultaneous detection with the multiplexed HT-OECT biosensor device 10.

Second, the multiplexed HT-OECT biosensor device 10 can be used for wearable biosensing applications. The multiplexed HT-OECT biosensor devices 10 can be first fabricated on ultrathin plastic or elastomer. The wavy structure of the source/drain electrodes 14, 16, used to increase the transconductance, also contributes to a higher stretchability on the elastomer. The flexible and miniature multiplexed HT-OECT biosensor devices 10 can be laminated on the skin for direct sweat analysis-the sweat flows into a microchannel through an inlet (which may be a dedicated inlet 60 or a microneedle 76) under external pressure and a capillary force (like a POC test on the skin) to flow over the gate electrode 18. Fluid can thus enter the HT-OECT biosensor device 10 from the external environment and come into contact with the gate electrode 18. Biomarkers in the sweat are captured by corresponding gate electrodes 18. The detected signal is then amplified by corresponding HT-OECT by causing a more pronounced change in the I_{ds}. The data can be collected by mobile phones via cable or wireless technology such as Bluetooth or the like.

The multiplexed HT-OECT biosensor devices 10 or sensors 74, 82 sensors described herein can be used for minimally-invasive biosensing applications such as that illustrated in FIGS. 5A, 5B, 6. To enable minimally-invasive detection with the multiplexed HT-OECT sensors, they may be integrated with microneedles 76 (FIGS. 5A, 5B), or catheters/probes 80 (FIG. 6) (e.g., brain probe). Microneedles 76 penetrate the skin to access the interstitial fluid (ISF) and capillary blood, with less or no pain. Bodily fluid in ISF or capillary blood can contact microneedles 76 or flow into the lumen of a hollow microneedle 76. When an antigen 34 such as a biomarker in the liquid pass the antibody-modified gate electrode 18 of the HT-OECT, they are captured, which, in turn, leads to a more pronounced change in the I_{ds} which can be measured and/or detected using external circuitry 38.

To integrated HT-OECT with medical catheters and brain probes, the multiplexed HT-OECT sensors 74, 82 can be fabricated on ultrathin plastic substrates 12 (such as parylene). The fabricated sensor devices 74, 82 can be wrapped on a medical catheter or laminated on a brain probe with biocompatible adhesive glues such as that illustrated in FIG. 6. The integration of multiplexed HT-OECT biosensors 82 with a medical catheter or probe 80 allows precise biomarker screening directly in blood vessels and ureter. Integration of multiplexed HT-OECT biosensors 82 with brain probes 80 allows directly biomarker-screening in brain cerebrospinal fluid, which is essential for a better understanding and treating brain cancer diseases.

The HT-OECT sensing elements or sensors can be fabricated on rigid, flexible, stretchable, and soft substrates 12. The CW configuration of the channel 30 that surrounds the circular gate electrode 18 with the source and drain electrodes 14, 16, allows a more uniform modulation of the V_{gs} to the I_{ds}. With the CW HT-OECT, the sensor design can be used in single and multiplexed sensors on a chip. The multiplexed HT-OECT sensors on a chip can be used for high-throughput biomarker screening applications. They can be used for POC tests at home, wearable tests on the skin (in sweat), and minimally-invasive tests with microneedles, catheters, and brain probes.

### Experimental section:

The PEDOT:PSS aqueous suspension (Clevios PH1000) was purchased from Heraeus Electronic Materials GmbH (Leverkusen, Germany). 99.5+% pure glycerol, dodecylbenzenesulfonic acid (DBSA), and glycidoxypropyltrimethoxysilane (GOPS) were bought from Sigma-Aldrich. The gold, silver, titanium (Ti), and palladium were provided by UCLA nano-lab. The photoresist SPR 700, MF 26A developer, and acetone for photolithography and patterning of electrodes 14, 16, 18 were provided by Integrated Systems Nanofabrication Cleanroom (ISNC) at California NanoSystems Institution (CNSI). PDMS (SYLGARD^{™} 184 Silicone Elastomer Kit) was purchased from Dow Inc. The antibody 32 for codeine 34 capturing was purchased from Base Pair Biotechnologies, Inc. Codeine was obtained from Spectrum Chemical Mfg. Corp, under a DEA license and with UCLA EHS Controlled Substances Use Authorization.

The Au electrodes 14, 16, 18 were deposited by e-beam evaporation using a CHA Solution Electron Beam Evaporator. The substrates 12 were first cleaned by acetone, IPA, and DI water. Then photoresist SPR 700 was spin-coated with 2000 rpm on substrates 12 using Headway spinner with PWM32 controller. Photolithography was performed to create patterns for the gate electrode 18, source electrode 14, and drain electrode 16. The exposed part was washed away with MF-26A developer. Then 10-nm-thick Ti and 100-nm-thick Au were deposited. The remaining photoresist was stripped off via acetone. The PDMS microfluidic channel (e.g., microfluidic chamber 54 and channel 56 as illustrated in intermediate PDMS layer 52 in FIG. 4) was fabricated in a 10:1 ratio with a laser-ablated PMMA mold (Universal Laser VLS 2.30). UV exposure was performed to enable bonding between PDMS and substrate 12 and top or cover 58.

The sequence of the antibody (aptamer) capture agent 32 for the antigen 34 codeine capturing is GGG ACA GGG CTA GCA GTA GGA TTG GGT GAG GGG ATG TGC TGT GGA GGC AAA GCT TCC G [SEQ ID NO: 1]. The powdered antibody 32 was first used to make an original solution in resuspension buffer (100 uM). Before using, the powder was de-frozen at room temperature (RT) for 5 min. The original solution was diluted with a folding buffer (2 uM). The diluted solution was then heated up 95 °C for 5 min and cooled down to RT. The, the antibody solution was mixed with the reducing buffer (1:1) and incubated for another 10 min. The reduced antibody solution was then diluted with PBS (w/ 1mM MgCl₂) to desired concentrations. Before modification, the Au gate electrode 18 was first cleaned with H₂SO₄ (0.5M), then cleaned with cyclic voltammetry (-0.4, 1.4 V) until the curves stabilize. Afterward, the electrodes 18 were rinsed with ethanol and dried with N₂ gun. The antibody buffer solution was then incubated on electrodes for 24 hrs. The mercaptoethanol (MCH) blocker solution (1 mM) with blocker 36 (MCH) was prepared by diluting the solution in PBS (w/ 1mM MgCl₂). The blocker solution was then incubated on the Au electrode 18 for 1 hour. Afterward, the electrodes 18 were rinsed with PBS solution and dried with N₂ gun.

The PEDOT:PSS suspension was obtained by mixing PEDOT:PSS, 1 v/v.% GOPS, 0.5 v/v.% DBSA, and 5 v/v.% glycerol. The mixture was further centrifuged to improve the homogeneity. Then the PEDOT:PSS suspension was coated on the substrate 12 deposited with source and drain electrodes 14, 16 and baked at 100 °C for 1 hour. The pattern of the channel 30 was defined by photolithography with SPR 700 photoresist. Acetone was used to develop the photoresist to minimize the damage to the electronic property of PEDOT:PSS channels. To measure the HT-OECT biosensor 10, the V_{gs} an V_{ds} are fixed constant (V_{gs}= 0.4 V, V_{ds}= -0.6 V).

## Claims

1. A multiplexed organic electrochemical transistor (OECT)-based biosensing device comprising:
a substrate;
one or more organic electrochemical transistor (OECT) sensors disposed on the substrate, wherein the one or more organic electrochemical transistor (OECT) sensors are specific to different biomarkers, each organic electrochemical transistor (OECT) sensor comprising:
a gate electrode disposed on the substrate or a separate substrate and having capture agents disposed thereon, the capture agents specific to one of the different biomarkers; and
source and drain electrodes disposed on the substrate and at least partially surrounding the gate electrode,
**characterized in that** the source and drain electrodes form an undulating pattern around the gate electrode with a channel formed between them.

2. The device of claim 1, wherein the substrate containing the one or more organic electrochemical transistor (OECT) sensors is/are located in a microfluidic device having an inlet and an outlet and a flow path containing the plurality of OECT sensors.

3. The device of claim 2, wherein the microfluidic device comprises a multilayered structure including one or more channels or chambers that comprise the flow path and a top or cover.

4. The device of claim 1, wherein the substrate and/or separate substrate is/are flexible or soft.

5. The device of claims 1, wherein the capture agents comprise one or more of: an antibody, aptamer, or nucleic acid.

6. The device of claim 1, further comprising external circuitry configured to apply a gate-source voltage and a drain-source voltage to the one or more organic electrochemical transistor (OECT) sensors and measure the drain-source current from the one or more organic electrochemical transistor (OECT) sensors.

7. The device of claim 1, wherein the channel(s) comprise poly(3,4-ethylenedioxythiophene) polystyrene sulfonate (PEDOT:PSS).

8. The device of claim 1, further comprising one or more additives in the channel(s), the additives selected from the group consisting of: dodecylbenzenesulphonic acid (DBSA), glycerol, ethylene glycol (EG), a fluorosurfactant, (3- glycidyloxypropyl)trimethoxysilane (GOPS).

9. The device of claim 4, further comprising a plurality of microneedles extending from the flexible substrate(s) and configured to deliver bodily fluids to the one or more organic electrochemical transistor (OECT) sensors.

10. The device of claim 4, wherein the organic electrochemical transistor (OECT)-based biosensor device has one or more inlets providing fluid access from an exterior environment to the one or more organic electrochemical transistor (OECT) sensors.

11. The device of claim 4, wherein the substrate comprises
an elongate catheter or shaft.

12. The device of claim 11, further comprising a plurality of multiplexed organic electrochemical transistor (OECT)-based biosensor(s) mounted thereon, each having respective gate electrodes with capture agents specific to different biomarkers.

13. The device of claim 11, wherein the one or more organic electrochemical transistor (OECT)-based biosensor(s) are mounted on a distal end of an intravascular catheter.

14. The device of claim 11, wherein the one or more organic electrochemical transistor (OECT)-based biosensor(s) are mounted on a brain probe.

## Patentansprüche

1. Eine auf einem organischen elektrochemischen Transistor (OECT) basierende Multiplex-Biosensorvorrichtung, die Folgendes umfasst:
ein Substrat;
einen oder mehrere organische elektrochemische Transistor (OECT)-Sensoren, die auf dem Substrat angeordnet sind, wobei der eine oder die mehreren organischen elektrochemischen Transistor (OECT)-Sensoren spezifisch für unterschiedliche Biomarker sind, wobei jeder organische elektrochemische Transistor (OECT)-Sensor Folgendes umfasst
eine Gate-Elektrode, die auf dem Substrat oder einem separaten Substrat angeordnet ist und auf der Einfangmittel angeordnet sind, wobei die Einfangmittel für einen der verschiedenen Biomarker spezifisch sind; und
Source- und Drain-Elektroden, die auf dem Substrat angeordnet sind und die Gate-Elektrode zumindest teilweise umgeben, **dadurch gekennzeichnet, dass** die Source- und Drain-Elektroden ein wellenförmiges Muster um die Gate-Elektrode herum bilden, wobei ein Kanal zwischen ihnen ausgebildet ist.

2. Die Vorrichtung nach Anspruch 1, wobei das Substrat, das den einen oder die mehreren organischen elektrochemischen Transistorsensoren (OECT-Sensoren) enthält, in einer mikrofluidischen Vorrichtung angeordnet ist/sind, die einen Einlass und einen Auslass und einen Strömungspfad aufweist, der die Vielzahl von OECT-Sensoren enthält.

3. Die Vorrichtung nach Anspruch 2, wobei die mikrofluidische Vorrichtung eine mehrschichtige Struktur mit einem oder mehreren Kanälen oder Kammern, die den Strömungsweg umfassen, und einer Oberseite oder Abdeckung umfasst.

4. Die Vorrichtung nach Anspruch 1, wobei das Substrat und/oder das separate Substrat flexibel oder weich ist/sind.

5. Die Vorrichtung nach Anspruch 1, wobei die Einfangmittel einen oder mehrere der folgenden Stoffe umfassen: einen Antikörper, ein Aptamer oder eine Nukleinsäure.

6. Die Vorrichtung nach Anspruch 1, die ferner eine externe Schaltung umfasst, die so gestaltet ist, dass sie eine Gate-Source-Spannung und eine Drain-Source-Spannung an den einen oder die mehreren organischen elektrochemischen Transistorsensoren (OECT) anlegt und den Drain-Source-Strom von dem einen oder den mehreren organischen elektrochemischen Transistorsensoren (OECT) misst.

7. Die Vorrichtung nach Anspruch 1, wobei der/die Kanal/Kanäle Poly(3,4-ethylendioxythiophen)-Polystyrolsulfonat (PEDOT:PSS) umfasst/umfassen.

8. Die Vorrichtung nach Anspruch 1, ferner umfassend ein oder mehrere Additive in dem/den Kanal/Kanälen, wobei die Additive ausgewählt sind aus der Gruppe bestehend aus: Dodecylbenzolsulfonsäure (DBSA), Glycerin, Ethylenglykol (EG), einem Fluortensid, (3-Glycidyloxypropyl)-Trimethoxysilan (GOPS).

9. Die Vorrichtung nach Anspruch 4, die ferner eine Vielzahl von Mikronadeln umfasst, die sich von dem/den flexiblen Substrat(en) aus erstrecken und so gestaltet sind, dass sie Körperflüssigkeiten an den einen oder die mehreren organischen elektrochemischen Transistorsensoren (OECT) abgeben.

10. Die Vorrichtung nach Anspruch 4, wobei die Biosensorvorrichtung auf Basis eines organischen elektrochemischen Transistors (OECT) einen oder mehrere Einlässe aufweist, die einen Flüssigkeitszugang von einer äußeren Umgebung zu dem einen oder den mehreren organischen elektrochemischen Transistorsensoren (OECT) bereitstellen.

11. Die Vorrichtung nach Anspruch 4, wobei das Substrat einen langgestreckten Katheter oder Schaft umfasst.

12. Die Vorrichtung nach Anspruch 11, die ferner eine Vielzahl von Multiplex-Biosensoren auf der Basis von organischen elektrochemischen Transistoren (OECT) umfasst, die darauf angebracht sind und jeweils entsprechende Gate-Elektroden mit für verschiedene Biomarker spezifischen Einfangmitteln aufweisen.

13. Die Vorrichtung nach Anspruch 11, wobei der/die organische(n) elektrochemische(n) Transistor (OECT)-Biosensor(en) an einem distalen Ende eines intravaskulären Katheters angebracht ist/sind.

14. Die Vorrichtung nach Anspruch 11, wobei der eine oder die mehreren Biosensoren auf der Basis eines organischen elektrochemischen Transistors (OECT) an einer Gehirnsonde angebracht sind.

## Revendications

1. Dispositif de biodétection à base de transistor électrochimique organique (OECT) multiplexé comprenant :
un substrat ;
un ou plusieurs capteurs à transistor électrochimique organique (OECT) disposés sur le substrat, dans lequel le ou les capteurs à transistor électrochimique organique (OECT) sont spécifiques à différents biomarqueurs, chaque capteur à transistor électrochimique organique (OECT) comprenant :
une électrode de grille disposée sur le substrat ou un substrat séparé et comportant des agents de capture disposés sur celle-ci, les agents de capture étant spécifiques de l'un des différents biomarqueurs ; et
des électrodes de source et de drain disposées sur le substrat et entourant au moins partiellement l'électrode de grille,
**caractérisé en ce que** les électrodes de source et de drain forment un motif ondulant autour de l'électrode de grille avec un canal formé entre elles.

2. Le dispositif selon la revendication 1, dans lequel le substrat contenant un ou plusieurs capteurs à transistor électrochimique organique (OECT) est/sont situé(s) dans un dispositif microfluidique doté d'une entrée et d'une sortie et d'une voie d'écoulement contenant la pluralité de capteurs OECT.

3. Le dispositif selon la revendication 2, dans lequel le dispositif microfluidique comprend une structure multicouche comportant un ou plusieurs canaux ou chambres qui comprennent la voie d'écoulement et une partie supérieure ou un couvercle.

4. Le dispositif selon la revendication 1, dans lequel le substrat et/ou le substrat séparé est/sont flexible(s) ou souple(s).

5. Le dispositif selon la revendication 1, dans lequel les agents de capture comprennent un ou plusieurs des éléments suivants : un anticorps, un aptamère ou un acide nucléique.

6. Le dispositif selon la revendication 1, comprenant en outre un circuit externe configuré pour appliquer une tension grille-source et une tension drain-source à un ou plusieurs capteurs à transistor électrochimique organique (OECT) et mesurer le courant drain-source à partir d'un ou plusieurs capteurs à transistor électrochimique organique (OECT).

7. Le dispositif selon la revendication 1, dans lequel le ou les canaux comprennent du poly (3,4-éthylène-dioxythiophène) polystyrène sulfonate (PEDOT:PSS).

8. Le dispositif selon la revendication 1, comprenant en outre un ou plusieurs additifs dans le(s) canal(aux), les additifs étant choisis dans le groupe consistant en : acide dodécylbenzènesulfonique (DBSA), glycérol, éthylène glycol (EG), un tensioactif fluoré, (3-glycidyloxypropyl)triméthoxysilane (GOPS).

9. Le dispositif selon la revendication 4, comprenant en outre une pluralité de micro-aiguilles s'étendant à partir du ou des substrats flexibles et configurées pour délivrer des fluides corporels à un ou plusieurs capteurs à transistor électrochimique organique (OECT).

10. Le dispositif selon la revendication 4, dans lequel le dispositif de biodétection à base de transistor électrochimique organique (OECT) comporte une ou plusieurs entrées permettant l'accès d'un fluide provenant d'un environnement extérieur à un ou plusieurs capteurs à transistor électrochimique organique (OECT).

11. Le dispositif selon la revendication 4, dans lequel le substrat comprend un cathéter ou une tige allongée.

12. Le dispositif selon la revendication 11, comprenant en outre une pluralité de biocapteurs multiplexés à base de transistors électrochimiques organiques (OECT) montés sur le substrat, chacun ayant des électrodes de grille respectives avec des agents de capture spécifiques à différents biomarqueurs.

13. Le dispositif selon la revendication 11, dans lequel le ou les biocapteurs à base de transistor électrochimique organique (OECT) sont montés sur une extrémité distale d'un cathéter intravasculaire.

14. Le dispositif selon la revendication 11, dans lequel le ou les biocapteurs à base de transistor électrochimique organique (OECT) sont montés sur une sonde cérébrale.
